## Europäisches Patentamt
## European Patent Office
## Office européen des brevets

(11) Veröffentlichungsnummer: **0 040 642**
**A1**

(12) **EUROPÄISCHE PATENTANMELDUNG**

(21) Anmeldenummer: **80102840.8**

(51) Int. Cl.³: **A 61 M 1/08, A 61 B 17/12**

(22) Anmeldetag: **22.05.80**

(43) Veröffentlichungstag der Anmeldung: **02.12.81**
**Patentblatt 81/48**

(71) Anmelder: **Huget, Richard, Neue Strasse 11, D-7901 Beimerstetten (DE)**

(84) Benannte Vertragsstaaten: **AT BE CH DE FR GB IT LI LU NL SE**

(72) Erfinder: **Huget, Richard, Neue Strasse 11, D-7901 Beimerstetten (DE)**

(54) **Geräte zur Induktion der Rückbildung von Neubildungen der Haut bzw. Schleimhaut, insbesondere Warzen und Kondylome.**

(57) Ein Gerät zur Induktion von Rückbildung von Warzen und Kondylomen besteht aus einem Ansaugtrichter (1) und einer darauf befestigten Ansaugvorrichtung (2). Dieses Gerät wird über der Warze bzw. Kondylom angesetzt und es wird Unterdruck über der behandelnden Stelle erzeugt. Diese Behandlung, die ca. 5–10 Minuten dauert, kann mehrmals wiederholt werden, führt nach ca. 2–4 Wochen zur vollständigen Rückbildung von so behandelten Warzen bzw. Kondylomen. Ein Gerät zur Rückbildung von Warzen an Fingern und Zehen besteht aus einem Ring aus elastischem Material (Gummi, Kunststoff). Er wird proximal zur Warze über den Finger (Zehe) gestülpt und für ca. 5–10 Minuten belassen. Der dadurch erzeugte venöse Stau führt zur vollständigen Rückbildung von so behandelten Warzen innerhalb 2–4 Wochen.

Geräte zur Induktion der Rückbildung von Neubildungen
der Haut bzw. Schleimhaut, insbesondere Warzen und Kondylome

Die Erfindung bezieht sich auf ein Gebiet der Medizin, das
unter den Begriff Hautkrankheiten, Virologie, fällt.

Warzen und Kondylome sind Neubildungen der Haut bzw.
Schleimhaut, die durch Viren erzeugt und durch Viren übertragen werden.
Je nach Lokalisation können sie zu erheblichen Beschwerden
führen, wie dies der Fall ist bei sogenannten Plantarwarzen
(Fußsohlewarzen) oder sie sind aus kosmetischen Gründen
lästig, wenn sie im Gesicht bzw. an Fingern lokalisiert
sind.

Kondylome sind lokalisiert an äußeren Geschlechtsteilen
des Mannes und der Frau und sind sehr infektiös und besonders lästig.

Die Behandlung von Warzen bzw. Kondylomen läßt sich in zwei
Gruppen einordnen: eine chirurgische und eine konservative.
Bei der chirurgischen Methode werden Warzen bzw. Kondylome
mechanisch entfernt. Die Wunde heilt ab, öfters mit Narbenbildung.
Bei der konservativen Therapie wird über die Warzen bzw.
Kondylome eine keratolytische Substanz, wie z. B. Milchsäure aufgetragen. Sie hat die Fähigkeit, die Oberfläche
der Warze derart aufzuweichen, daß sie die anschließende
mechanische Entfernung möglich macht.
Wird auf die mechanische Entfernung verzichtet, so muß die
Substanz längere Zeit - wochenlang angewendet werden. Neuerdings werden den keratolytischen Substanzen, Substanzen mit
virostatischen Eigenschaften beigemengt.

Der Erfindung liegt folgende Beobachtung zugrunde: Neubildungen der Haut bzw. Schleimhaut wie Warzen und Kondylome
bilden sich zurück, wenn über der behandelten Stelle ein Unterdruck erzeugt wird.

Warzen bilden sich auch dann zurück, wenn in ihrer unmittelbaren Umgebung ein venöser Stau induziert wird.

Diese Beobachtung läßt sich mit Geräten, die der Gegenstand der Erfindung sind, in die Praxis umsetzen.

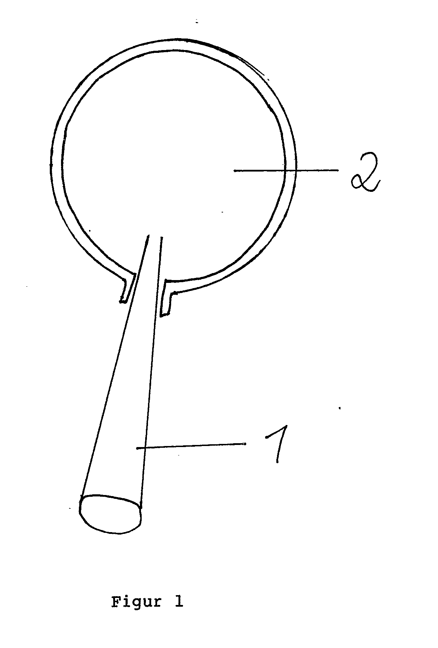

Das Gerät zur Induktion von Rückbildung der Neubildungen der Haut bzw. Schleimhaut, insbesondere Warzen und Kondylome besteht aus zwei Teilen:     Figur 1

1.    Einem Ansaugtrichter (1), der über die behandelte Stelle aufgesetzt wird

2.    Einer Ansaugvorrichtung (2), die ihrerseits auf den Ansaugtrichter aufgesetzt wird.

Die einzige Aufgabe der Ansaugvorrichtung ist, über dem Ansaugtrichter einen Unterdruck zu erzeugen. Am zweckmäßigsten wird dazu ein hohler Gummiball mit Fassung für diesen Ansaugtrichter verwendet. Vor dem Aufsetzen auf die behandelte Stelle wird der Gummiball mit der Hand zusammengedrückt, das Gerät auf die Haut um die Warze aufgesetzt und der Gummiball losgelassen.

Durch den dadurch entstandenen Unterdruck saugt sich das Gerät an der Haut fest.

Es kann auch eine andere Ansaugvorrichtung als ein Gummiball verwendet werden, z. B. ein Zylinder mit beweglichem Kolben, der manuell oder durch eine unter Spannung stehende Feder bewegt wird.

Es können verschiedene Ansaugtrichter verwendet werden. Sie sind untereinander austauschbar. Das ermöglicht solche Ansaugtrichter zu verwenden, die sich den örtlichen Bedingungen der Haut, auf der die Warzen bzw. Kondylome liegen am besten eignen.

Die Behandlung wird so durchgeführt, daß das Gerät auf die zu behandelnde Stelle aufgesetzt wird und ein Unterdruck erzeugt wird.

Das Gerät bleibt auf der behandelnden Stelle 5 - 10 Minuten lang und wird danach entfernt. Die Behandlung kann mehrmals täglich wiederholt werden. Sie ist auch dann wirksam, wenn sie nur 2 - 3/Wochen durchgeführt wird. Die behandelten Warzen bzw. Kondylome bilden sich innerhalb 2 - 4 Wochen zurück, ohne Narben zu hinterlassen, wobei je öfter die Behandlung durchgeführt wird, desto schneller bilden sich die Neubildungen zurück.

Das Gerät zur Induktion der Rückbildung von Warzen durch Erzeugung von lokalem venösem Stau wird durch einen Ring gebildet, der aus weichem elastischem Material (Gummi bzw. Kunststoff) besteht und der über den von Warzen befallenen Finger bzw. Zehe gestülpt wird.

Die Behandlung mit dem Ring wird so durchgeführt, daß der Ring nach Anlegen proximal zur Warze in einer Entfernung von ca. 0,5 - 1 cm, für ca. 5 - 10 Minuten belassen wird. Die Behandlung kann mehrmals täglich wiederholt werden. Der Druck, der durch das Anlegen des Ringes erzeugt wird, muß ausreichen, um eine venöse Stauung in unmittelbarer Umgebung der Warze hervorzurufen. Das wird erkennbar an rasch einsetzender blauer Verfärbung der Haut um die Warze herum.

So behandelte Warzen bilden sich (je nach Größe) nach 2 - 4 Wochen zurück, ohne Narben zu hinterlassen.

Die Vorteile, die die Behandlung von Warzen bzw. Kondylomen mit diesen Geräten bietet, sind folgende. Die Behandlung ist schmerzlos. Dadurch, daß die Geräte in keinen unmittelbaren Kontakt mit den Warzen bzw. Kondylomen kommen, fällt das Risiko der Übertragung der Neubildungen auf die gesunde Haut weg, wie z. B. bei der chirurgischen Methode. Es besteht auch nicht die Gefahr, daß die gesunde Haut um die Warze herum geschädigt wird, wie das der Fall sein kann bei Verwendung von keratolytischen Substanzen. Die Dauer der Behandlung bis zur Heilung bei mehrmaliger täglicher Anwendung ist kürzer als bei der konservativen Therapie. Es bleiben keine Narben, der Erfolg der Behandlung ist dauerhaft. Ein weiterer Vorteil der Behandlung bei Verwendung dieser Geräte liegt in seiner Einfachheit.

Patentansprüche

1.  Ein Gerät zur Induktion der Rückbildung der Neubil-
    dungen der Haut und Schleimhaut, insbesondere Warzen
    und Kondylome, der dadurch gekennzeichnet ist, daß
    mittels der angewandten Geräte über den Warzen bzw.
    Kondylomen ein Unterdruck erzeugt wird.

2.  Verfahren zur Herstellung eines Geräte nach Patentanspruch 1, der dadurch gekennzeichnet ist, daß das
    Gerät zur Erzeugung des Unterdrucks aus zwei Teilen
    besteht, die untereinander ausgetauscht werden können:
    einem Ansaugtrichter und einer Ansaugvorrichtung.

3.  Verfahren zur Herstellung eines Gerätes nach Patentanspruch 1, der dadurch gekennzeichnet ist, daß dieses
    Gerät zur Erzeugung des Unterdrucks ein in sich unteilbares Gerät ist, das heißt, der Ansaugtrichter und die
    Ansaugvorrichtung können nicht voneinander getrennt
    werden.

4.  Ein Gerät zur Induktion der Rückbildung von Warzen an
    Fingern bzw. Zehen, der dadurch gekennzeichnet ist, daß
    ein Ring *oder Binde)* aus elastischem Material (Gummi, Kunststoff) *oder gewickelt*
    proximal zur Warze über den Finger, Zehe, gestülpt
    wird und in unmittelbarer Umgebung der Warze ein ve-
    nöser Stau induziert wird.

0040642

1/1

Figur 1

# EUROPÄISCHER TEILRECHERCHENBERICHT,

der nach Regel 45 des Europäischen Patentübereinkommens für das weitere Verfahren als
europäischer Recherchenbericht gilt

Europäisches Patentamt

004064 2 Nummer der Anmeldung

EP 80102840.8

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X | FR - A - 774 601 (D.CLERGUE) <br> + Gesamt + <br> -- | 2 |
| X | CH - A - 281 903 (NORRIE WELLS) <br> + Gesamt + <br> ---- | 3 |

**KLASSIFIKATION DER ANMELDUNG (Int. Cl. )**

A 61 M 1/08
A 61 B 17/12

**RECHERCHIERTE SACHGEBIETE (Int. Cl. )**

A 61 M 1/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 2,3
Unvollständig recherchierte Patentansprüche: —
Nicht recherchierte Patentansprüche: 1,4
Grund für die Beschränkung der Recherche:

Artikel 52(4) EPÜ

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

| Recherchenort | ßdatum der Recherche | Prüfer |
|---|---|---|
| WIEN | 20-01-1981 | LUDWIG |

EPA Form 1505.1  06.78